# EUROPÄISCHE PATENTSCHRIFT

(11) **EP 1 357 883 B1**
(45) Veröffentlichungstag und Bekanntmachung des Hinweises auf die Patenterteilung: **29.12.2004**
(21) Anmeldenummer: 02716064.7
(22) Anmeldetag: 08.01.2002
(51) Int. Cl.: A61K 6/087

(54) **KATIONISCH HÄRTBARE DENTALMASSEN**
CATIONICALLY CURABLE DENTAL MATERIALS
PATES DENTAIRES DURCISSABLES DE MANIERE CATIONIQUE

(30) Priorität: 09.01.2001 DE 10100680
(43) Veröffentlichungstag der Anmeldung: 05.11.2003
(73) Patentinhaber: 3M ESPE AG, 82229 Seefeld (DE)
(72) Erfinder: HOESCHELER, Stefan, 82211 Herrsching (DE); WEINMANN, Wolfgang, 82362 Weilheim (DE); STIPPSCHILD, Andrea, 86899 Landsberg (DE); WEGNER, Susanne, 82211 Herrsching (DE)
(74) Vertreter: Brem, Roland
(86) Internationale Anmeldenummer: PCT/EP2002/000101
(87) Internationale Veröffentlichungsnummer: WO 2002/055028

(56) Entgegenhaltungen:
- EP-A- 0 728 790
- WO-A-00/20494

## Beschreibung

Die Erfindung betrifft polymerisierbare Dentalmassen auf der Basis von kationisch härtbaren Monomeren sowie ein Verfahren zu deren Herstellung.

Polymerisierbare Dentalmassen auf der Basis von kationisch härtbaren Monomeren, wie beispielsweise Kunststofffüllungs- und Befestigungsmaterialien, sind bereits bekannt. Sie enthalten neben einer kationisch härtbaren Monomer-Verbindung ein Initiatorsystem, das geeignet ist, die kationische Polymerisation zu starten, und Füllstoffe sowie eventuell Verzögerer, Beschleuniger und Hilfsstoffe. Die Monomere zusammen mit dem Initiatorsystem sowie gegebenenfalls Verzögerem, Beschleunigem und Hilfsstoffen wird auch Matrix genannt, in die die Füllstoffe eingearbeitet sind.

Bevorzugt sind Füllstoffe, die eine Röntgensichtbarkeit der Dentalmassen gewährleisten. Aus diagnostischer Sicht ist es vorteilhaft, wenn die üblicherweise zahnfarbene Dentalmasse röntgendiagnostisch von der natürlichen Zahnsubstanz unterscheidbar ist. Das bedeutet, dass die Dentalmasse für Röntgenstrahlen deutlich schlechter zu durchdringen sein sollte, als die umgebende Zahnsubstanz.

Um ferner eine besonders transluzente Dentalmasse zu erhalten, werden vorzugsweise solche Füllstoffe eingesetzt, deren Brechungsindex im Bereich desjenigen der Matrix liegt. Üblicherweise liegen diese Brechungsindices bei n_{D} = 1,49 - 1,54.

Bekannte Dentalmassen enthalten beispielsweise Quarz als Füllstoff. Damit lässt sich eine gute mechanische Belastbarkeit des Materials realisieren. Zur Anwendung in Dentalmassen ist Quarz als alleiniger Füllstoff nur bedingt geeignet, da die damit gefüllten Dentalmassen keine ausreichende Röntgensichtbarkeit aufweisen. Insbesondere bei der Verwendung von Epoxiden als Matrix ist Quarz ungeeignet, da er mit 1,55 einen zu hohen Brechungsindex besitzt.

Polymerisierbare Dentalmassen auf der Basis von Epoxiden und deren Verwendung als Füllungs- und Befestigungsmäterialien, werden ausführlich in der WO 98/22521 A1 beschrieben. Dabei ist der Polymerisationsschrumpf, der bisher bei polymerisierbaren Dentalmassen auf der Basis von Acrylaten und/oder Methacrylaten unvermeidlich auftrat, von besonderer Bedeutung. Wünschenswert sind Dentalmassen, die während der Polymerisation exakt formtreu bleiben oder einen Volumenschrumpf von kleiner 1 % aufweisen.

In der DE 198 49 388 A1 wird als möglicher Füllstoff ein bariumfreies röntgenopakes Dentalglas beschrieben, das einen SiO₂- Gehalt von 20 bis 45 Gew.-% aufweist. Dementsprechend sind in diesem Füllstoff mindestens 65% andere Oxide enthalten. Aufgrund des Maximalgehalts von 10 Gew.-% La₂O₃ und 10 Gew.-% ZrO₂ weist die Glaszusammensetzung einen relativ hohen Gehalt an basischen und/oder amphoteren Oxiden auf. Diese Füllstoffe werden vor allem zur Herstellung von Dentalmassen auf der Basis von radikalisch härtbaren Monomeren eingesetzt. Bei der Verwendung von derartigen Gläsern als Füllstoff in kationisch härtbaren Dentalmassen, härtet die Masse nicht mehr ausreichend aus bzw. polymerisiert überhaupt nicht. Die resultierende Dentalmasse besitzt nur ungenügende mechanische Festigkeitswerte.

Ein in der EP 0 634 373 A1 beschriebenes röntgensichtbares Dentalglas enthält beispielsweise 15-35 Gew.-% SrO, 0-10 Gew.-% CaO, 5 - 20 Gew.-% B₂O₃, 5 -20 Gew.-% Al₂O₃ und 45 - 65 Gew.-% SiO₂. Die hergestellten Gläser weisen zwar gute Röntgensichtbarkeit auf. Nachteilig ist jedoch der zu hohe Gehalt von zusammen mindestens 10 Gew.-% Al₂O₃ und B₂O₃ sowie die geringe Lagerstabilität der mit diesem Füllstoff hergestellten Dentalmassen.

Die US 4,764,497 beschreibt SiO₂-haltige Füllstoffe sowie deren Herstellungsverfahren. Diese Füllstoffe werden nach einem Sol-Gel-Verfahren hergestellt, um möglichst sphärische Partikel zu erhalten. Sie werden beispielsweise verwendet, um die mechanischen Eigenschaften und die Oberflächenbeschaffenheit von Dentalmassen zu verbessern und das Material zu verstärken. Es ist offenbart, diese Füllstoffe in radikalisch härtende Acrylat- bzw. Methacrylat-Matrices einzuarbeiten.

In der WO 00/20494 A1 wird eine röntgenopake kationisch polymerisierbare Zusammensetzung mit radioopaken Füllstoffen beschrieben. Sie enthält neben einer kationisch härtbaren Monomer-Verbindung Füllstoffe und ein Initiatorsystem, das geeignet ist, die kationische Polymerisation zu starten. Die genannten Füllstoffe sind derart ausgewählt, dass die Barcol-Härte der polymerisierten Dentalmasse innerhalb von 30 min mindestens 10 Einheiten erreicht. Die Dentalmassen mit den beschriebenen Füllstoffen weisen in der Regel eine nicht ausreichende Lagerstabilität oder zu geringe Reaktivität auf.

Mit den aus dem Stand der Technik bekannten Füllstoffen war es bisher nicht möglich, auf der Basis der hier beschriebenen kationisch härtbaren Monomere lagerstabile und gleichzeitig reaktive Dentalmassen herzustellen.

Es ist daher die Aufgabe der vorliegenden Erfindung, eine kationisch härtbare röntgenopake Dentalmasse zur Verfügung zu stellen, deren Lagerstabilität und Reaktivität den allgemeinen Anforderungen entspricht.

Dies wird erreicht durch eine Dentalmasse, umfassend:
(a) 3 bis 80 Gew.-% eines oder mehrerer kationisch härtbarer Monomere,
(b) 3 bis 90 Gew.-% eines oder mehrerer röntgenopaker Füllstoffe,
(c) 0,01 bis 25 Gew.-% initiatoren, Verzögerem und/oder Beschleunigern,
(d) 0 bis 25 Gew.-% Hilfsstoffen,
wobei die Prozentangaben jeweils auf das Gesamtgewicht der Masse bezogen sind und wobei der Füllstoff (b) durch ein Schmelzverfahren bei mindestens 1550°C hergestellt wird und derart ausgewählt ist, dass er mindestens 65 Gew.-% SiO₂ enthält und einen Brechungsindex von n_{D} = 1,49 - 1,54 aufweist, und dass die nach Methode 4 (siehe Beschreibung zu den Beispielen) gemessenen Werte der Viskositätbestimmung der polymerisierbaren Dentalmasse bei einer Lagertemperatur von 20 bis 25 °C über mindestens 9 Monate einen Wert von +/-50% des Ausgangswerts, gemessen 24 h nach Herstellung der polymerisierbaren Dentalmasse, nicht über- bzw. unterschreitet, und dass die polymerisierbare Dentalmasse eine derartige Reaktivität aufweist, dass nach dem Polymerisationsstart der Betrag des von der Dentalmasse erzeugten maximalen Wärmeflusses, der nach Methode 5 (siehe Beschreibung zu den Beispielen) bestimmt wird, mindestens 0,8 mW/mg beträgt und dieser maximale Wärmefluss innerhalb von höchstens 60 s erreicht wird.

Eine derartige Dentalmasse enthält eines oder mehrere kationisch härtbare Monomere sowie Initiatoren und gegebenenfalls Verzögerer, Beschleuniger und/oder Hilfsstoffe, die die sogenannte Matrix darstellen. In diese Matrix sind der oder die Füllstoffe eingebettet. Die Füllstoffe haben bei der Verwendung in Dentalmassen die Aufgabe, bestimmte physikalische Eigenschaften der Dentalmasse einzustellen. Dabei kann der Füllstoffanteil 3 - 90 Gew.-% betragen. Er liegt üblicherweise bei 20 - 90 Gew.-%, besonders bevorzugt bei 40 - 85 Gew.-%.

Durch die erfindungsgemäßen Dentalmassen mit der beschriebenen Zusammensetzung können folgende Anforderungen erfüllt werden:

### Röntgensichtbarkeit:

Die Röntgensichtbarkeit von dentalen Füllstoffen wird relativ zur Röntgenabsorption von Aluminium nach EN ISO 4049, Abschnitt 7.14 angegeben. Die gemäß der vorliegenden Erfindung beanspruchten Massen weisen ein relatives Aluminiumröntgenäquivalent von größer als 140% auf.

### Ästhetik/Opazität:

Um Dentalmassen zu erhalten, deren Ästhetik bzw. Transluzenz der natürlichen Zahnsubstanz nahekommt, dürfen die Brechungsindices von Matrix und Füllstoffen nur wenig von einander abweichen. Üblicherweise sollen die Brechungsindices (n_{D}) beider Komponenten weniger als 0,05 von einander differieren. Zur Beurteilung der ästhetischen Eigenschaften dient die Opazität, die einen Wert von 40-70% annehmen soll und von den erfindungsgemäßen Dentalmassen erreicht wird.

### Mechanische Belastbarkeit:

Die mechanische Belastbarkeit einer Dentalmasse sowie die Abrasionsresistenz muss so hoch sein, dass die Dentalmasse den beim Kauen auftretenden Belastungen mehrere Jahre widerstehen kann. Zur Bestimmung der mechanischen Belastbarkeit werden vor allem die Biegefestigkeit und der E-Modul (EN ISO 4049), Druckfestigkeit, Oberflächenhärte und die Zwei- bzw. Drei-Körper-Abrasion (J Dent. 1994;22 Suppl 1:S21-7) bestimmt.

Bisher waren keine kationisch härtbaren Dentalmassen bekannt, die röntgensichtbare Füllstoffe enthielten, den ästhetischen Anforderungen genügten und sowohl lagerstabil als auch ausreichend reaktiv waren. Die erfindungsgemäßen Dentalmassen erfüllen diese Anforderungen. Überraschenderweise wurde festgestellt, dass sich mit diesen Dentalmassen zugleich gute mechanische Festigkeitswerte erreichen lassen. Die erfindungsgemäßen Massen weisen eine Biegefestigkeit von mehr als 80 MPa auf.

Als kationisch härtbare Monomere werden beispielsweise die in der WO 98/22521 A1, der WO 00/20494 A1 sowie der EP 0 897 710 A2 beschriebenen Verbindungen, Oxetane, Vinylether, Spiroorthoester, Spiroorthocarbonate bicyclische Orthoester, bicyclische Monolactone, bicyclische Bislactone, cyclische Carbonate oder Kombinationen hiervon eingesetzt. Es ist ebenso möglich, kationisch härtbare Monomere einzusetzen, die zusätzlich radikalisch härtende Gruppen aufweisen.

Initiatoren gemäß Komponente (c) der erfindungsgemäßen Massen können sein: Lewis- oder Broensted-Säuren bzw. Verbindungen, die solche Säuren freisetzen, welche die Polymerisation initiieren, beispielsweise BF₃ oder dessen etherische Addukte (BF₃.THF, BF₃.Et₂O, etc.), AlCl₃, FeCl₃, HPF₆, HAsF₆, HSbF₆, HBF₄ oder Substanzen, die nach Bestrahlen durch UV oder sichtbares Licht oder durch Wärme und/oder Druck die Polymerisation auslösen, wie (eta-6-Cumol)(eta-5-cyclopentadienyl)eisen-hexafluorophosphat, (eta-6-Cumol)(eta-5-cyclopenta-dienyl)eisen-tetrafluoroborat, (eta-6-Cumol)(eta-5-cyclopentadienyl)eisen-hexa-fluoroantimonat, substituierte Diaryliodoniumsalze und Triarylsulfoniumsalze. Besonders bevorzugt können zusammengesetzte Initiatorsysteme, wie in der US 6,084,004 oder der US 5,545,676 beschrieben eingesetzt werden. Ferner können Kombinationen der verschiedenen Bestandteile als Initiatorsystem eingesetzt werden.

Als Beschleuniger gemäß Komponente (c) können kondensierte Polyaromaten, Peroxyverbindungen vom Typ der Perester, der Diacylperoxide, der Peroxydicarbonate und der Hydroperoxide eingesetzt werden. Bevorzugt werden Hydroperoxide verwendet, als besonders bevorzugter Beschleuniger kommt Cumolhydroperoxid in etwa 70 bis 90 %-iger Lösung in Cumol zum Einsatz. Als Verzögerer können Basen, typischerweise tertiäre Amine, zugesetzt werden.

Die Komponente (c) liegt in der erfindungsgemäßen Dentalmasse in einer Menge von 0,01 bis 25 Gew.-%, vorzugsweise 0,01 bis 20 Gew.-%, bezogen auf das Gesamtgewicht der Masse, vor.

Geeignete Hilfsstoffe gemäß Komponente (d) können beispielsweise übliche auf dem Dentalgebiet eingesetzte Stabilisatoren, Rheologiemodifikatoren, Pigmente und/oder Verdünnungsmittel sein.

Die röntgenopaken Füllstoffe nach Komponente (b) sind ausgewählt aus der Gruppe der röntgensichtbaren Gläser mit angepasstem Brechungsindex. Hierzu eignen sich SiO₂- Gläser, die eines oder mehrere Oxide der Elemente der 5. und 6. Periode, wie Sr, Y, Zr, Nb, Ba, La, Hf, Ta und/oder eines oder mehrere Oxide anderer schwerer Elemente, wie Zn, Ga, Ge, enthalten. Prinzipiell sind die Oxide der genannten Elemente, aber auch deren Carbonate, Hydroxide, Silicate, Borate oder andere Glasrohstoffe für die Herstellung der Füllstoffe (b) geeignet. Die Berechnung erfolgt ungeachtet der eingesetzten Rohstoffe immer bezogen auf die oxidische Form.

Es ist ein Gegenstand der vorliegenden Erfindung, dass solche Gläser als Füllstoffe eingesetzt werden, die durch ein Schmelzverfahren bei mindestens 1550°C hergestellt sind und derart ausgewant sind, dass sie mindestens 65 Gew.-% SiO₂ enthalten und einen Brechungsindex von n_{D} = 1,49 - 1,54 aufweisen.

Ferner weisen die erfindungsgemäßen Dentalmassen mit den ausgewählten Füllstoffen eine gute Lagerstabilität auf. Dies zeigt sich durch die nach Methode 4 (siehe Beschreibung zu den Beispielen) gemessenen Werte der Viskositätbestimmung. Diese Werte liegen bei einer Lagertemperatur von 20 bis 25 °C über mindestens 9 Monate bei höchstens +/- 50% des Ausgangswerts, gemessen 24 h nach Herstellung der polymerisierbaren Dentalmasse.

Besonders vorteilhaft ist es, wenn die Werte der Viskositätsbestimmung bei einer Lagertemperatur von 20 bis 25 °C über mindestens 15 Monate +/- 50 %, insbesondere +/- 30 % des Ausgangswerts nicht über- bzw. unterschreiten.

Die Reaktivität der erfindungsgemäßen polymerisierbaren Dentalmassen mit dem Füllstoff nach Komponente (b) ist so hoch, dass nach dem Polymerisationsstart der Betrag des von der Dentalmasse erzeugten maximalen Wärmeflusses, der nach Methode 5 bestimmt wird, mindestens 0,8 mW/mg beträgt und dieser maximale Wärmefluss innerhalb von höchstens 60 s erreicht wird.

Die Füllstoffe nach Komponente (b) werden auf folgenden Wegen hergestellt:

Es wird ein Hochtemperaturschmelzverfahren durchgeführt, bei dem die Anwesenheit von Oxiden, die geeignet sind, die Schmelzeigenschaften der Gläser zu begünstigen, nicht erforderlich ist. Derartige Hochtemperaturschmelzverfahren sind beispielsweise Induktionsschmelz-verfahren. Bei diesen Verfahren werden die Bestandteile des Füllstoffs in einem Tiegel aufgeschmolzen, ohne dass die Tiegelwand mit der heißen Schmelze in Berührung gerät. Dies erreicht man durch Erhitzen der Glasrohstoffe in einem elektrischen Induktionsfeld. Es entsteht dabei ein thermischer Gradient im Inneren des Tiegels, so dass die zum Schmelzen benötigte Temperatur nur im Kern erreicht wird. Übliche Schmelztemperaturen liegen bei 1500 - 3000 °C. Auch bei Plasmaschmelzverfahren kommt man ohne Verwendung von Oxiden, die die Schmelzeigenschaften begünstigen, aus. Hierbei werden die Glasrohstoffe in einem elektrischen Lichtbogen aufgeschmolzen. Es können noch höhere Temperaturen als beim Induktionsschmelzverfahren erreicht werden. Ein weiterer Vorteil einiger Plasmaschmelzverfahren besteht in den extremen Abkühlraten. Somit können auch Gläser, die sehr stark zur Phasenseparation neigen, klar erschmolzen werden. Besonders vorteilhaft ist es, dass bei einigen Plasmaschmelzverfahren das Glas bereits in einer pulverförmigen Form anfällt, so dass sich der nachfolgende Mahlvorgang weniger aufwendig gestaltet. Weitere Verfahren zum Hochtemperaturschmelzen sind möglich. Dies sind beispielsweise verschiedene Lichtbogenschmelzverfahren von oxidischen Werkstoffen, wie sie aus der SiO₂-Glasherstellung sowie der schmelzgegossenen Feuerfeststeine für die Glasindustrie bekannt sind. Weitere Hochtemperaturschmelzverfahren sind z.B. Laserschmelzen, bei denen sehr hohe Temperaturen erzielt werden, wie in R. Harrysson: Glass formation in the System Y₂O₃ - Al₂O₃ - SiO₂ under Conditions of LASER Melting beschrieben.

Die vorliegend beanspruchten Füllstoffe weisen eine Liquidustemperatur höher als 1500 °C, vorzugsweise höher 1600 °C auf. Um eine genügend geringe Viskosität der Schmelze zu erhalten, sind Temperaturen von mindestens 50 °C oberhalb der Liquidustemperatur erforderlich. Bei den beanspruchten Füllstoffen handelt es sich daher um Systeme, die bei einer Temperatur von mindestens 1550 °C, vorzugsweise 1650 °C geschmolzen werden. Bei dieser Temperatur erhält man verhältnismäßig hochviskose Schmelzen. Es können auch Schmelzen von niedrigerer Viskosität hergestellt werden, die bei Schmelztemperaturen von 1600 °C, vorzugsweise 1700 °C vorliegen.

Füllstoffe, die zur Verwendung in den erfindungsgemäßen Dentalmassen geeignet sind, weisen bevorzugt folgende Zusammensetzung auf:

| **Oxide** | **Anteil** |
|---|---|
| SiO₂ | 65 -95 Gew.-% vorzugsweise 75 - 90 Gew% |
| Al₂O₃, B₂O₃ und/oder P₂O₅ | insgesamt 0 - 3 Gew.-% vorzugsweise 0 - Gew% |
| MgO und/oder CaO | insgesamt 0 - 10 Gew.-% vorzugsweise 0 - 3 Gew% |
| eines oder mehrere der Oxide ausgewählt aus der Gruppe: Li₂O, Na₂O, K₂O, Rb₂O, Cs₂O | insgesamt 0 - 3 Gew.-% vorzugsweise 0 - 1 Gew% |
| SrO und/oder BaO | insgesamt 0 - 3 Gew.-% vorzugsweise 0 - 2 Gew% |
| eines oder mehrere der Oxide ausgewählt aus der Gruppe Y₂O₃, Ln₂O₃, ZrO₂, HfO₂, Nb₂O₅, Ta₂O₅, SnO₂, GeO₂, In₂O₃, WO₃ | insgesamt 5 - 35 Gew.-% vorzugsweise 10 - 25 Gew% |

Wobei Ln ein Element der Lanthanoidengruppe (La - Lu) bedeutet und wobei sowohl die kristallwasserfreien als auch die kristallwasserhaltigen Oxide eingesetzt werden können.

Die mittlere Komgröße wird mit einem Lasergranulometer (Fa. Cilas) bestimmt. Besonders vorteilhaft ist es, die Füllstoffe mit einer mittleren Komgröße vorzugsweise von 0,6 bis 3,0 µm besonders bevorzugt von 0,8 bis 1,5 µm und einer spezifischen Oberfläche (BET) von bevorzugt 1- 35 m²/g, besonders bevorzugt 3 - 9 m²/g, einzuarbeiten.

Diese Zusammensetzungen stellen Füllstoffe dar, die einen Brechungsindex passend zur Matrix besitzen und die Röntgensichtbarkeit der Dentalmasse gewährleisten.

Überraschenderweise wurde festgestellt, dass die erfindungsgemäßen Dentalmassen die oben genannten Vorteile aufweisen und zusätzlich eine Biegefestigkeit von mindesten 80 MPa besitzen. Gleichzeitig erhält man so kationisch härtende Dentalmassen, die eine geeignete Reaktivität besitzen und gleichzeitig in Pastenform gut zu lagern sind.

Im folgenden ist die Herstellung von verschiedenen Gläsern zur Verwendung als Füllstoffe für polymerisierbare Dentalmassen auf der Basis von kationisch härtbaren Monomeren an Hand von Beispielen beschrieben.

Tabelle 1 zeigt die oxidische Zusammensetzung der verschiedenen Gläser. Die Gläser nach den Beispielen 1-23 werden mittels nachfolgend beschriebener Verfahren hergestellt:

### 1. Glasherstellung

### Beispiele 1 - 9 (Schmelzverfahren):

Als Ausgangsstoffe werden käufliche Oxide, wie Siliziumdioxid, Lanthanoxid, Zirkonoxid, Yttriumoxid, Calziumoxid, Hafniumdioxid und Tantaloxid verwendet. Die gewünschten Oxide werden entsprechend dem in der Glaszusammensetzung gewünschten Verhältnis homogen gemischt. Das erhaltene Pulver wird dann nach einem der nachstehend beschriebenen Schmelzverfahren zu einem Glas weiterverarbeitet:

### a) Plasma-Schmelzverfahren (Beispiele 1-2):

Das vorstehend erhaltene Pulver wird in ein Plasmaschmelzaggregat überführt und bei einer Temperatur von 2000 - 3000 °C geschmolzen.

### b) Induktions-Schmelzverfahren (Beispiele 3-9):

Das aus dem Gemisch der Oxide erhaltene Pulver wird in einem induktiv beheizten Ofen bei einer Temperatur von 1550 - 2800 °C über eine Zeit von 5 - 60 min erschmolzen und anschließend in Wasser abgeschreckt.

Ferner können anstatt der hier beschriebenen Plasma- oder Induktions-Schmelzverfahren auch weitere Schmelzverfahren, bei denen entsprechend hohe Temperaturen von mindestens 1550 °C erreicht werden, angewendet werden. Dies sind beispielsweise Laser-Schmelzverfahren.

### Vergleichsbeipiele 10 - 15 (Sol-Gel-Verfahren):

Zur Glasherstellung gelangen metallorganische Verbindungen wie Tetraethylorthosilikat, Aluminiumbutoxid, Zirkonpropoxid, Lanthanethoxid, Calciumisopropoxid zur Anwendung. Die Rohstoffe werden in Isopropanol gelöst und unter tropfenweiser Zugabe von Wasser hydrolysiert. Nach mehrstündigem Rühren gelieren die Lösungen, die nach Abfiltrieren im Trockenschrank ca. 24 h bei 150 °C getrocknet und anschließend zermörsert werden. Das erhaltenene Pulver wird 2 h bei einer Temperatur von 800 - 1050 °C kalziniert.

### Vergleichsbeipiele 16 - 17 (Sol-Gel-Verfahren):

Die Vergleichsbeispiele 16 und 17 sind dem Stand der Technik entnommen. Die Herstellung ist auf Seite 67 und Seite 69 der WO 00/20494 A1 unter "Example 25" bzw. "Example 33" beschrieben.

### Vergleichsbeipiele 18 - 26 (Schmelzverfahren):

Unter Verwendung von Glasrohstoffen wie Quarzmehl, Aluminiumhydroxid, Mullit, Natriumcarbonat, Borsäure, Zirkonoxid, Lanthanoxid, Yttriumoxid, Calciumhydroxid, Magnesiumoxid, Lithiumcarbonat werden nach herkömmlichen Verfahren Gläser im Platintiegel bei einer Temperatur von 1400 bis 1500 °C über eine Zeit von 2 - 4 h geschmolzen und anschließend in Wasser abgeschreckt. Zur Glasherstellung können prinzipiell verschiedenste Rohstoffe, wie Carbonate, Hydroxide, Oxide und Silikate eingesetzt werden.

### Vergleichsbeipiele 27 - 29 (Schmelzverfahren):

Die Vergleichsbeispiele 27 bis 29 sind nach den in der WO 00/20494 beschriebenen Verfahren hergestellt. Sie sind dort als "Example 6", "Example 11" und als "Example 16" aufgeführt.

### Vergleichsbeipiel 30:

Der Füllstoff besteht aus einem kommerziell erhältlichen, pyrogen erzeugten SiO₂ (Aerosil® OX 50, Fa. Degussa).

### Vergleichsbeipiel 31:

Das Vergleichsbeispiel 31 stellt einen kommerziell erhältlichen Quarz (Fa. Quarzwerke Frechen) dar.

### 2. Mahlvorgang:

Die gemäß den oben beschriebenen Beispielen 1 - 31 erhaltenen Glaspulver werden, mit Ausnahme der plasmaerschmolzenen Gläser (Beispiele 1 und 2) sowie des in Beispiel 30 beschriebenen pyrogen erzeugten SiO₂ auf eine geeignete mittlere Komgröße mittels herkömmlichen Mahlverfahren gemahlen.

Die Vormahlung der Glaspulver zu einer mittleren Komgröße von 10 - 50 µm erfolgt in einer Scheibenschwingmühle (Fa. Siebtechnik; Mahldauer 10 - 15 min). Bei den plasmageschmolzenen Proben erhält man bereits aufgrund des Schmelzverfahrens Glasgranulate von einer mittleren Korngröße von ca. 50 µm, so dass hier der Arbeitsschritt der Vormahlung entfallen kann. Die Feinmahlung wird bei allen Glaspulvern anschließend in einem mit Aluminiumoxid ausgekleideten Attritor mit Zirkonoxidmahlkugeln einer Größe von 0,8 mm durchgeführt. Als Mahlmedium wird Wasser verwendet.

Aus den Gläsern 18 bis 21 werden jeweils zwei Pasten mit unterschiedlicher Komverteilung hergestellt. Dazu werden die Gläser der Beispiele 18 bis 21 als Ausgangsmaterial für zwei unterschiedliche Mahlprozesse verwendet. Für die Weiterverarbeitung zu den Pasten P 24, P 26, P 28, P 30 werden die Gläser der Beispiele 18 bis 21 sowohl vor- als auch feingemahlen, gemäß den oben beschriebenen Verfahren. Ohne Vormahlung werden die Gläser der Beispiele 18 bis 21 für die Weiterverarbeitung zu den Pasten P 25, P 27, P 29, P 31 eingesetzt. Hierbei erfolgt nur eine Vormahlung mit einer Scheibenschwingmühle über einen längeren Zeitraum (60 min) ohne anschließende Feinmahlung. Die resultierende mittlere Komgröße ist gröber.

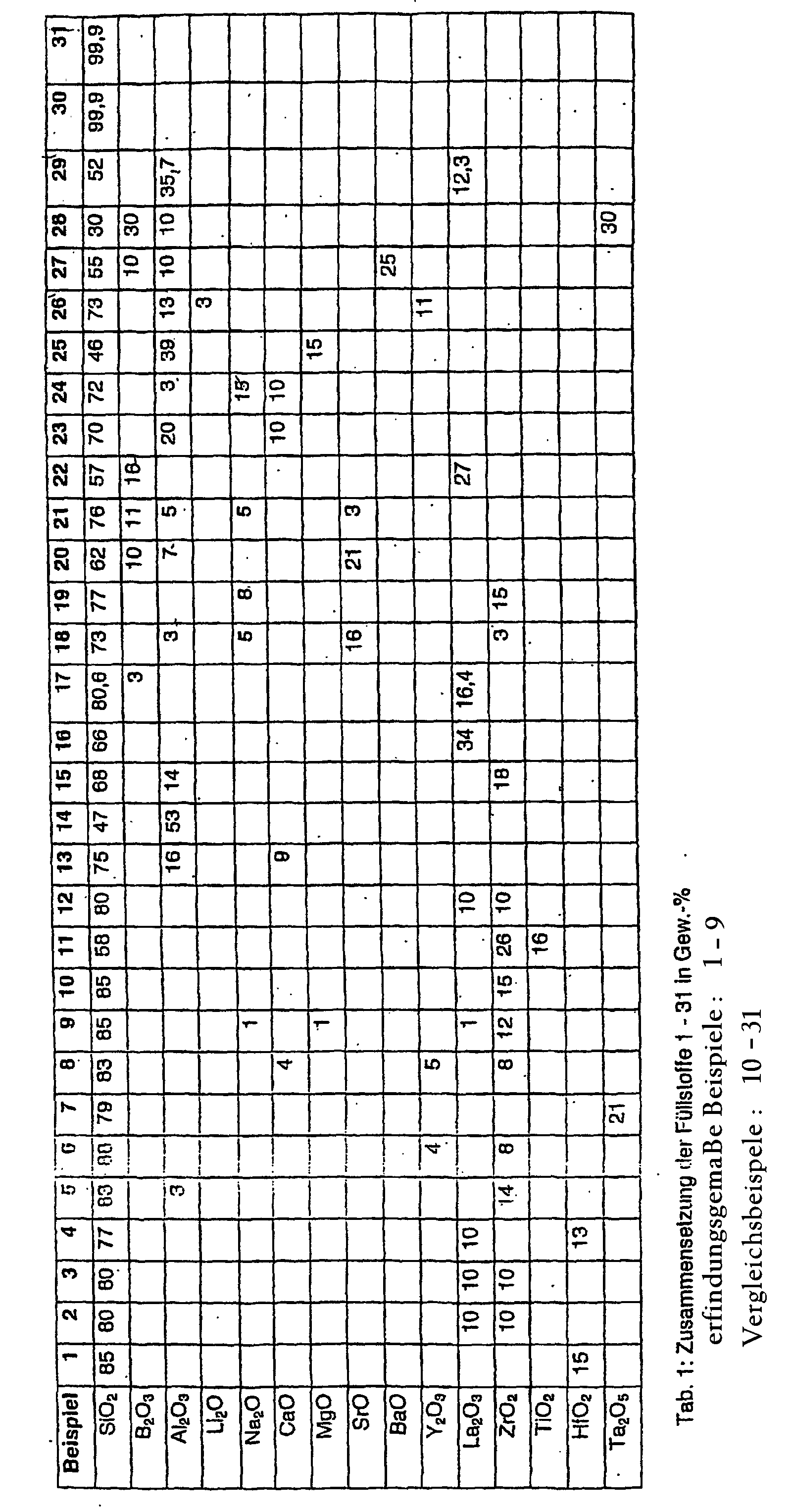

In der nachfolgenden Tabelle 2 ist der Brechungsindex der einzelnen Füllstoffe der Tabelle 1 angegeben. Zur Bestimmung des Brechungsindex werden die zu untersuchenden Glaspulver in Flüssigkeiten gegeben, deren Brechungsindex bekannt ist. In einem Lichtmikroskop läßt sich so der Brechungsindex des jeweiligen Füllstoffes exakt messen.

**Tabelle 2:**

| **Beispiel** | **Brechungsindex** | **Beispiel** | **Brechungsindex** |
|---|---|---|---|
| 1 | 1,530 | 17 | 1,505 |
| 2 | 1,527 | 18 | 1,518 |
| 3 | 1,528 | 19 | 1,538 |
| 4 | 1,526 | 20 | 1,513 |
| 5 | 1,514 | 21 | 1,481 |
| 6 | 1,511 | 22 | 1,55 |
| 7 | 1,539 | 23 | 1,481 |
| 8 | 1,525 | 24 | 1,504 |
| 9 | 1,502 | 25 | 1,540 |
| 10 | 1,529 | 26 | 1,527 |
| 11 | 1,72 | 27 | 1,528 |
| 12 | 1,529 | 28 | 1,514 |
| 13 | 1,491 | 29 | 1,528 |
| 14 | 1,484 | 30 | 1,465 |
| 15 | 1,549 | 31 | 1,545 |
| 16 | 1,522 | | |

### 3. Oberflächenbehandlung:

Die Glaspulver werden zur einfacheren Weiterverarbeitung in acetonischer Lösung silanisiert. In allen Beispielen gelangt Glycidyloxypropyltrimethoxysilan zur Anwendung. Folgende Silankonzentrationen in Gew.-% bezogen auf den Füllstoff werden eingesetzt:

| | |
|---|---|
| Beispiel 1-29, 31 | 3 Gew% |
| Beispiel 24 - 27 | 1 Gew% |
| Beispiel 30 | 6 Gew% |

Weitere Methoden zur Oberflächenbehandlung sind ebenfalls denkbar.

### 4. Weiterverarbeitung zu Dentalmassen:

Nach geeigneter Oberflächenbehandlung werden zur Herstellung von Dentalmassen die Füllstoffe mittels eines handelsüblichen Kneters in eine Monomermatrix eingearbeitet. Die Monomermatrix weist beispielsweise eine der folgenden Zusammensetzungen auf:

| **Matrix 1:** | |
|---|---|
| 14,53 Gew.-% | Di - (3-epoxycyclohexylethyl)methylphenylsilan |
| 14,53 Gew.-% | 1,3,5,7-Tetrakis(2,1-ethandiyl-3,4-epoxycyclohexyl)-1,3,5,7-tetramethyltetraethylsiloxan |
| 0,30 Gew.-% | Campherchinon |
| 0,10 Gew.-% | Ethyl-4-dimethylaminobenzoat |
| 0,54 Gew.-% | 4-Methylphenyl-4-isopropylphenyl-iodoniumtetrakis (pentafluorophenyl)borat |
| 70,00 Gew.-% | Füllstoff |

| **Matrix 2:** | |
|---|---|
| 19,10 Gew.-% | Di - (3-epoxycyclohexylethyl)methylphenylsilan |
| 9,50 Gew.-% | 1,3,5,7-Tetrakis(2,1-ethandiyl-3,4-epoxycyclohexyl)-1,3,5,7-tetramethyltetraethylsiloxan |
| 0,40 Gew.-% | Campherchinon |
| 0,15 Gew.-% | Ethyl-4-dimethylaminobenzoat |
| 0,72 Gew.-% | 4-Methylphenyl-4-isopropylphenyl-iodoniumtetrakis(pentafluorophenyl)borat |
| 70,13 Gew.-% | Füllstoff |

| **Matrix 3:** | |
|---|---|
| 12,42 Gew.-% | Di - (3-epoxycyclohexylethyl)methylphenylsilan |
| 16,10 Gew.-% | 1,3,5,7-Tetrakis(2,1-ethandiyl-3,4-epoxycyclohexyl)-1,3,5,7-tetramethyltetraethylsiloxan |
| 0,35 Gew.-% | Campherchinon |
| 0,12 Gew.-% | Ethyl-4-dimethylaminobenzoat |
| 0,80 Gew.-% | 4-Methylphenyl-4-isopropylphenyl-iodoniumtetrakis(pentafluorophenyl)borat |
| 70,21 Gew-% | Füllstoff |

| **Matrix 4:** | |
|---|---|
| 35,5 Gew.-% | Di - (3-epoxycyclohexylethyl)methyl phenyl silan |
| 35,5 Gew.-% | 1,3,5,7-Tetrakis(2,1-ethandiyl-3,4-epoxycyclohexyl)-1,3,5,7-tetramethyltetraethylsiloxan |
| 0,90 Gew.-% | Campherchinon |
| 0,25 Gew.-% | Ethyl-4-dimethylaminobenzoat |
| 0,45 Gew.-% | 4-Methylphenyl-4-isopropylphenyl-iodoniumtetrakis(pentafluorophenyl)borat |
| 27,40 Gew-% | Füllstoff |

Die Charakterisierung der Pasten erfolgt hinsichtlich folgender Prüfkriterien, die in der Tabelle 3 zusammengefaßt sind:

### Opazität (Methode 1)

Die Opazität wird an 3,6 mm dicken Proben bestimmt. Hierzu werden Probenplättchen hergestellt und von jeder Seite 40 s mit einem Lichtpolymerisationsgerät (Elipar®, Fa. ESPE) polymerisiert. Die Opazitätsmessung wird an einem handelsüblichen Meßgerät (Labscan, Fa. CieLab) durchgeführt.

### Polymerisationstiefe (Methode 2)

In einer zylindrischen Probenform (Höhe 12 mm, Durchmesser 7 mm) werden Prüfkörper hergestellt, die 40 s mit einem Lichtpolymerisationsgerät (Elipar ®, Fa. ESPE) von einer Seite belichtet werden. Nach dem Belichten werden von dem Zylinder auf der lichtabgewandten Seite mit einem Messer die unpolymerisierten Bestandteile abgeschnitten. Die verbleibende Zylinderlänge, die aus nicht schneidbarer ausgehärteter Dentalmasse besteht, beschreibt die Polymerisationstiefe.

### Dreipunktbiegefestigkeit (Methode 3)

Dreipunktbiegefestigkeit nach EN ISO 4049: Die Biegefestigkeit wird nach EN ISO 4049 an 2x2x25 mm Stäbchen bestimmt. Sie wird als Maß für eine ausreichende Polymerisation herangezogen. Für einen Einsatz als Dentalmasse sollte eine Biegefestigkeit von 80 MPa vorzugsweise 100 MPa erreichbar sein.

### Lagerstabilität (Methode 4)

Die Lagerstabilität wird durch Viskositätsmessung über die Verformung einer unpolymerisierten Kugel aus Dentalmasse bestimmt. Dazu wird eine Pastenkugel mit einer Masse von 0,4 g hergestellt. Die Kugel wird 60 s lang zwischen zwei Folien mit einer Masse von 1575 g belastet, und anschließend die Höhe des verformten Prüfkörpers gemessen. Für die Beurteilung der Lagerstabilität wird der Startwert, der 24 h nach Herstellung der Dentalmasse gemessen wird, der 9 Monatswert und der 15 Monatswert herangezogen. Als lagerstabil gelten Massen, deren Messwert nach 9 Monaten den Startwert um nicht mehr als 50 % über- bzw. unterschreitet.

### Röntgenopazität:

Die Röntgensichtbarkeit wird relativ zur Röntgenabsorption von Aluminium nach EN ISO 4049, Abschnitt 7.14 gemessen.
Die nachstehende Tabelle 3 zeigt die Eigenschaften der verschiedenen nach den erfindungsgemäßen Zusammensetzungen hergestellten Dentalmassen im Vergleich zu solchen Dentalmassen, die aus dem Stand der Technik bekannte Füllstoffe enthalten
Es bedeuten in der Tabelle 3:
- Ausg. :: Ausgehärtet während der Lagerung
- n. c. :: no cure - keine Aushärtung möglich

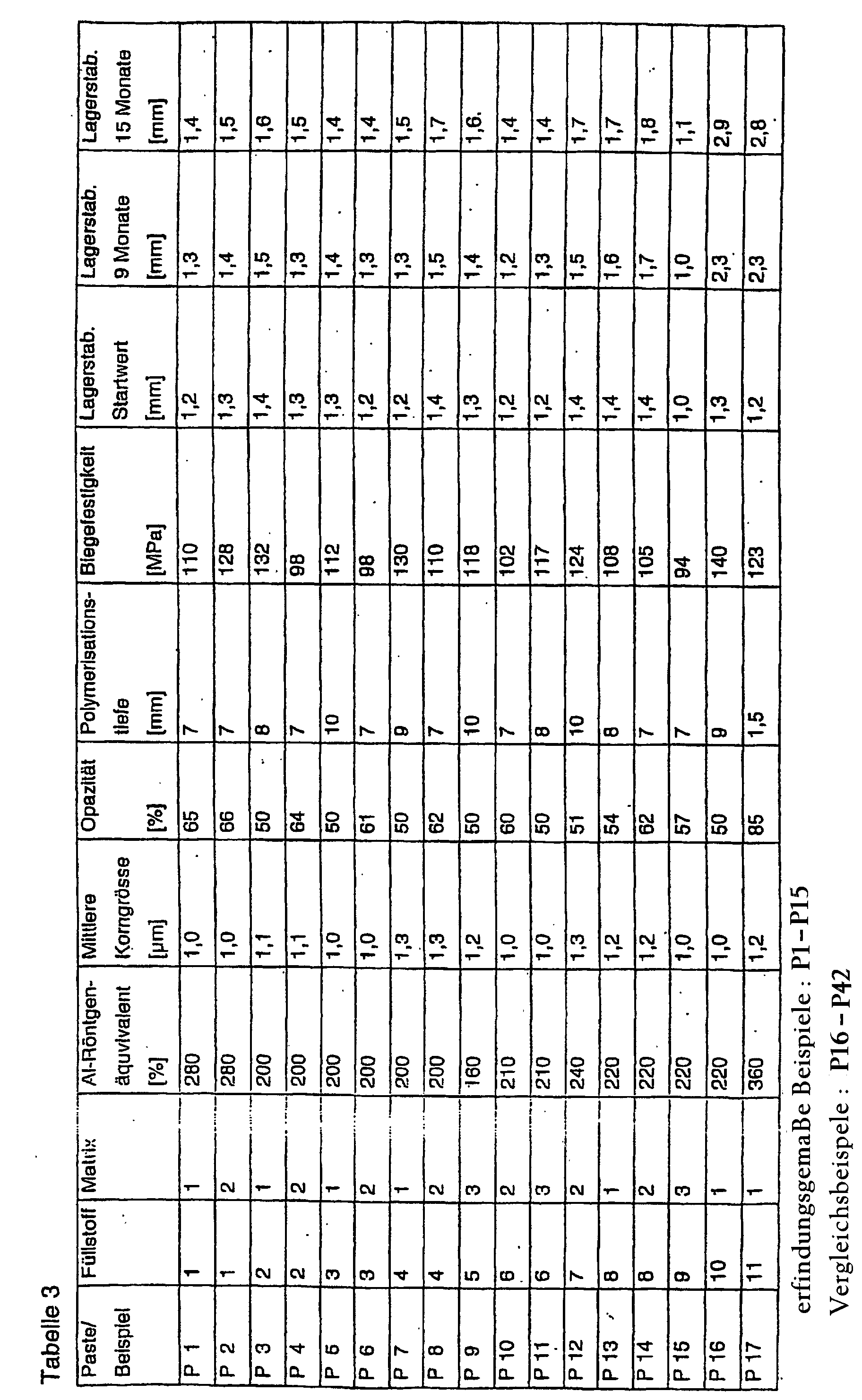

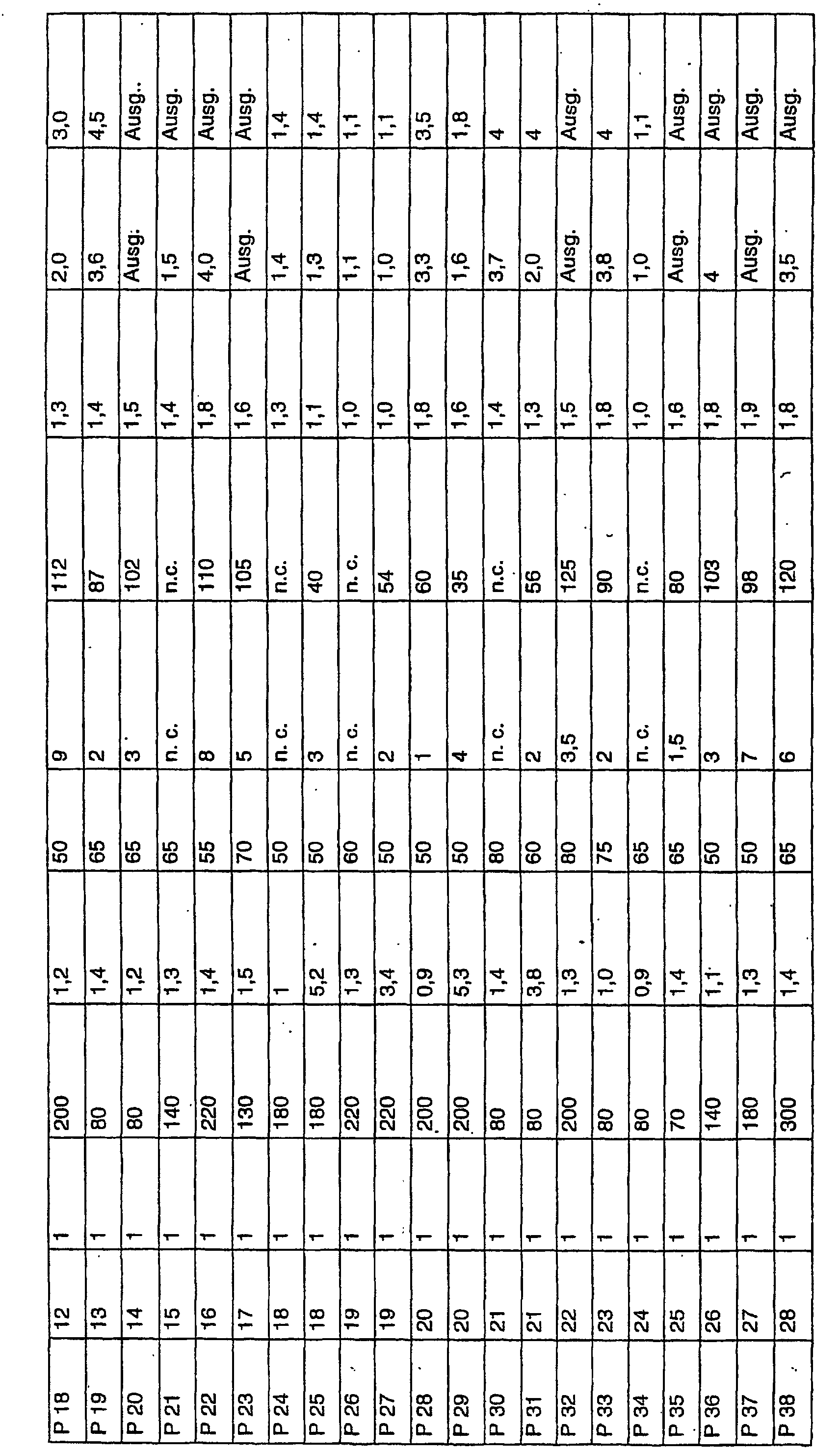

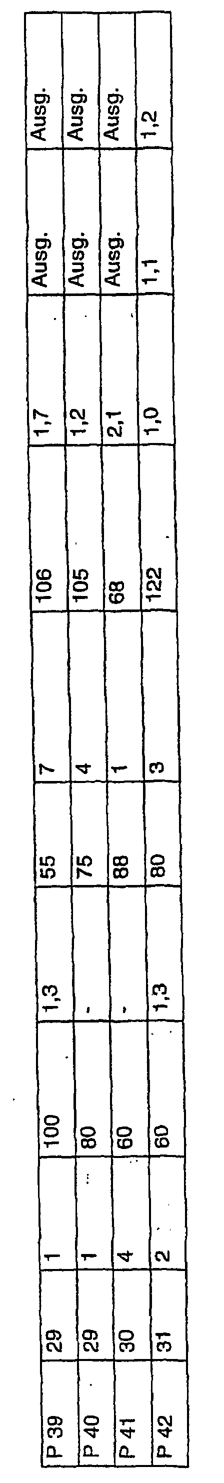

Es ist zu erkennen, dass die erfindungsgemäßen Dentalmassen P1 - P15 gegenüber den aus dem Stand der Technik bekannten Dentalmassen P16 -P42 deutlich bessere Werte für die Lagerstabilität aufweisen. Quarz und pyrogene Kieselsäuren als Füllstoffe (P41, P42) sind nicht röntgensichtbar.

### Reaktivität (Methode5):

Dentalmassen müssen in kurzer Zeit verarbeitbar und benutzbar sein. Insbesondere gilt das für Füllungsmaterialien, die mit Licht gehärtet werden und sofort nach der klinischen Versorgung mechanisch belastbar sein sollen. Wie aus der Tabelle 4 hervorgeht, gelingt dies mit den erfindungsgemäßen reaktiven Massen (Beispiel P1 - P15), die mit den geeigneten Füllstoffen versehen sind. Die aus dem Stand der Technik bekannten Füllstoffe, die zu lagerstabilen Dentalmassen führen, zeigen bei der kationischen Polymerisation eine zu geringe Reaktivität (z.B. P27, P29). Die Reaktivität der Massen wird per Photo-DSC Messung bestimmt. Hierzu werden etwa 30 mg der Masse in einem DSC-Messgerät (Fa. Netzsch DSC 200 cell) mit einer Polymerisationlampe (Elipar, Fa. ESPE Dental AG) 40 s belichtet und der Wärmefluss ab Belichtungsbeginn zeitlich verfolgt. Reaktive Massen im Sinne der vorliegenden Erfindung liegen vor, wenn der Betrag des von der Masse erzeugten Wärmeflusses mindestens 0,8 mW/mg ist und der maximale Wärmefluss innerhalb von höchstens 60 s erreicht wird.

**Tabelle 4**

| Paste Nr. | Betrag des maximalen Wärmefluss [mW/mg] | Betrag des maximalen Wärmefluss wird erreicht nach [s] |
|---|---|---|
| P 1 . | 1,8 | 21 |
| P 3 | 1,9 | 18 |
| P 6 | 1,5 | 20 |
| P 7 | 2,1 | 16 |
| P 15 | 1,1 | 34 |
| P 27 | 0,4 | 50 |
| P 29 | 0,6 | 72 |

## Patentansprüche

1. Polymerisierbare Dentalmasse, enthaltend
(a) 3 bis 80 Gew.-% eines oder mehrerer kationisch härtbarer Monomere,
(b) 3 bis 90 Gew.-% eines oder mehrerer röntgenopaker Füllstoffe,
(c) 0,01 bis 25 Gew.-% Initiatoren, Verzögerer und/oder Beschleuniger,
(d) 0 bis 25 Gew.-% Hilfsstoffe,
wobei die Prozentangaben jeweils auf das Gesamtgewicht der Masse bezogen sind und wobei der Füllstoff (b) durch ein Schmelzvervahren bei mindestens 1550°C hergestellt ist und derart ausgewählt ist, dass er mindestens 65 Gew.-% SiO₂ enthält und einen Brechungsindex von n_{D} =1,49 - 1,54 aufweist,
und dass die Viskosität der polymerisierbaren Dentalmasse bei einer Lagertemperatur von 20 bis 25 °C über mindestens 9 Monate einen Wert von +/- 50% des Ausgangswerts aufweist, gemessen 24 h nach Herstellung der polymerisierbaren Dentalmasse,
und dass die polymerisierbare Dentalmasse eine derartige Reaktivität aufweist, dass nach dem Polymerisationsstart der Betrag des von der Dentalmasse erzeugten maximalen Wärmeflusses, mindestens 0,8 mW/mg beträgt und dieser maximale Wärmefluss innerhalb von höchstens 60 s erreicht wird.

2. Polymerisierbare Dentalmasse nach Anspruch 1, wobei der Füllstoff (b) einen Brechungsindex von n_{D} = 1,50 - 1,53 aufweist.

3. Polymerisierbare Dentalmasse nach Anspruch 1 oder 2, wobei der Füllstoff (b) mindestens 65 Gew.-% SiO₂, insgesamt höchstens 35 Gew.-% eines oder mehrerer der Oxide der Elemente der 5. und/oder 6. Periode, insgesamt höchstens 3 Gew.-% eines oder mehrerer der Oxide der ersten Hauptgruppe und insgesamt höchstens 3 Gew.-% der Oxide B₂O₃, Al₂O₃, P₂O₅ enthält

4. Polymerisierbare Dentalmasse nach einem der Ansprüche 1 bis 3, wobei der Füllstoff (b) mindestens 65 Gew.-% SiO₂, insgesamt höchstens 35 Gew.-% eines oder mehrere der Oxide, ausgewählt aus der Gruppe Y₂O₃, Lanthanidoxide, ZrO₂, HfO₂, Nb₂O₅, Ta₂O₅, In₂O₃, SnO₂, WO₃, insgesamt höchstens 3 Gew.-% eines oder mehrerer der Oxide der ersten Hauptgruppe und insgesamt höchstens 3 Gew.-% der Oxide B₂O₃, Al₂O₃, P₂O₅ enthält.

5. Polymerisierbare Dentalmasse nach einem der Ansprüche 1 bis 4, wobei der Füllstoff (b) derart ausgewählt ist, dass er eine Rontgenopazität aufweist, die einem Aluminiumröntgenäquivalent von mindestens 100% entspricht.

6. Polymerisierbare Dentalmasse nach einem der Ansprüche 1 bis 5, wobei der Füllstoff (b) nach einem Hochtemperatur-Schmelzverfahren hergestellt ist

7. Polymerisierbare Dentalmasse nach Anspruch 6, wobei das Hochtemperatur-Schmelzverfahren bei mindestens 1600°C durchgeführt wird.

8. Polymerisierbare Dentalmasse nach einem der Ansprüche 1 bis 7, wobei der Füllstoff (b) eine mittlere Komgrösse von 0,1 - 15 µm und eine spezifische Oberfläche von 1- 35 m²/g aufweist.

9. Polymerisierbare Dentalmasse nach einem der Ansprüche 1 bis 8, wobei der Füllstoff (b) eine mittlere Komgrösse von 1 - 4 µm und eine spezifische Oberfläche von 3-9 m²/g aufweist.

10. Verfahren zur Herstellung einer polymerisierbaren Dentalmasse, bei dem der Füllstoff durch ein Hochtemperaturschrmelzverfahren bei mindestens 1550°C hergestellt und in eine Matrix, enthaltend
(i) kationisch härtbare Monomere
(ii) Initiatoren, Verzögerer, Beschleuniger und/oder Hilfsstoffe eingearbeitet wird,
wobei der Füllstoff mindestens 65 Gew.-% SiO₂ enthält.

11. Verfahren zur Herstellung einer polymerisierbaren Dentalmasse nach Anspruch 10, bei dem
(a) der Füllstoff aus den einzelnen Komponenten durch ein Schmelzverfahren hergestellt wird,
(b) der geschmolzene Füllstoff auf eine Komgröße von 0,1 -15 µm gemahlen wird
(c) der unter (b) erhaltene Füllstoff oberflächenbehandelt wird und
(d) der oberflächenbehandelte Füllstoff in eine Matrix, enthaltend
(i) kationisch härtbare Monomere
(ii) Initiatoren, Verzögerer, Beschleuniger und/oder Hilfsstoffe
eingearbeitet wird.

12. Verfahren nach einem der Ansprüche 10 bis 11 bei dem das Schmelzverfahren bei einer Temperatur von mindestens 1600°C durchgeführt wird.

13. Verfahren nach einem der Ansprüche 10 bis 12, bei dem das Schmelzverfahren in einem induktiv beheizten Ofen bei 1550-3000°C über 5 bis 60 Minuten oder in einem Ptasmaschmetzaggregat bei 2000-3000°C durchgeführt wird.

14. Verfahren nach einem der Ansprüche 11 bis 13, bei dem der oberflächenbehandelte Füllstoff zu einer Dentalmasse verarbeitet wird, enthaltend
(a) 3 bis 80 Gew.-% eines oder mehrerer kationisch härtbarer Monomere,
(b) 3 bis 90 Gew.-% des oberflächenbahandelten Füllstoffes,
(c) 0,01 bis 25 Gew.-% Initiatoren, Verzögerer und/oder Beschleuniger,
(d) 0 bis 25 Gew.-% Hilfsstoffe,
wobei die Prozentangaben jeweils auf das Gesamtgewicht der Masse bezogen sind und wobei der Füllstoff (b) einen Brechungsindex von n_{D} = 1,49 - 1,54 aufweist,
und die Visikosität der polymerisierbaren Dentalmasse bei einer Lagertemperatur von 20 bis 25 °C über mindestens 9 Monate einen Wert von +/- 50% des Ausgangswerts aufweist, gemessen 24 h nach Herstellung der polymerisierbaren Dentalmasse,
und die Dentalmasse eine derartige Reaktivität aufweist, dass nach dem Polymerisationsstart der Betrag des von der Dentalmasss erzeugten maximalen Wärmeflusses mindestens 0,8 mW/mg beträgt und dieser maximale Wärmefluss innerhalb von höchstens 60 s erreicht wird.

15. Polymerisierbare Dentalmasse, hergestellt nach einem Verfahren gemäß den Ansprüchen 10 bis 14.

16. Verwendung einer polymerisierbaren Dentalmasse nach einem der Ansprüche 1 bis 9 oder 15 als dentales Füllungsmaterial, Bondingmaterial oder Befestigungsmaterial.

## Claims

1. Polymerizable dental material, comprising:
(a) 3 to 80 weight% of one or more cationically curable monomers,
(b) 3 to 90 weight% of one or more X-ray-opaque fillers,
(c) 0.01 to 25 weight% of initiators, retarders and/or accelerators,
(d) 0 to 25 weight% of auxiliary agents,
wherein the percentages are in each case with reference to the total weight of the material and wherein the filler (b) is prepared by a melting process at at least 1550°C and is chosen so that it comprises at least 65 weight% of SiO₂ and exhibits a refractive index of n_{D} = 1.49-1.54,
and so that the viscosity of the polymerizable dental material, at a storage temperature of 20 to 25°C over at least 9 months, exhibits a value of +/-50% of the starting value, measured 24 h after preparation of the polymerizable dental material,
and so that the polymerizable dental material exhibits a reactivity such that, after the start of polymerization, the amount of the maximum heat flux generated by the dental material amounts to at least 0.8 mW/mg and this maximum heat flux is achieved within at most 60 s.

2. Polymerizable dental material according to Claim 1, wherein the filler (b) exhibits a refractive index of n_{D} = 1.50-1.53.

3. Polymerizable dental material according to Claim 1 or 2, wherein the filler (b) comprises at least 65 weight% of SiO₂, a total of at most 35 weight% of one or more of the oxides of the elements from the 5th and/or 6th period, a total of at most 3 weight% of one or more of the oxides from the first main group and a total of at most 3 weight% of the oxides B₂O₃, Al₂O₃ and P₂O₅.

4. Polymerizable dental material according to one of Claims 1 to 3, wherein the filler (b) comprises at least 65 weight% of SiO₂, a total of at most 35 weight% of one or more of the oxides chosen from the group Y₂O₃, lanthanide oxides, ZrO₂, HfO₂, Nb₂O₅, Ta₂O₅, In₂O₃, SnO₂ and WO₃, a total of at most 3 weight% of one or more of the oxides from the first main group and a total of at most 3 weight% of the oxides B₂O₃, Al₂O₃ and P₂O₅.

5. Polymerizable dental material according to one of Claims 1 to 4, wherein the filler (b) is chosen so that it exhibits an X-ray opacity corresponding to an aluminium X-ray equivalent of at least 100%.

6. Polymerizable dental material according to one of Claims 1 to 5, wherein the filler (b) is prepared according to a high temperature melting process.

7. Polymerizable dental material according to Claim 6, wherein the high temperature melting process is carried out at at least 1600°C.

8. Polymerizable dental material according to one of Claims 1 to 7, wherein the filler (b) exhibits an average particle size of 0.1-15 µm and a specific surface of 1-35 m²/g.

9. Polymerizable dental material according to one of Claims 1 to 8, wherein the filler (b) exhibits an average particle size of 1-4 µm and a specific surface of 3-9 m²/g.

10. Process for the preparation of a polymerizable dental material, which comprises preparing the filler by a high temperature melting process at at least 1550°C and incorporating it in a matrix comprising
(i) cationically curable monomers
(ii) initiators, retarders, accelerators and/or auxiliary agents,
the filler comprising at least 65 weight% of SiO₂.

11. Process for the preparation of a polymerizable dental material according to Claim 10, wherein
(a) the filler is prepared from the individual components by a melting process,
(b) the molten filler is ground to a particle size of 0.1-15 µm,
(c) the filler obtained in (b) is surface-treated, and
(d) the surface-treated filler is incorporated in a matrix comprising
(i) cationically curable monomers
(ii) initiators, retarders, accelerators and/or auxiliary agents.

12. Process according to one of Claims 10 to 11, wherein the melting process is carried out at a temperature of at least 1600°C.

13. Process according to one of Claims 10 to 12, wherein the melting process is carried out in an inductively heated furnace at 1550-3000°C over 5 to 60 minutes or in a plasma melting unit at 2000-3000°C.

14. Process according to one of Claims 11 to 13, wherein the surface-treated filler is processed to a dental material comprising
(a) 3 to 80 weight% of one or more cationically curable monomers,
(b) 3 to 90 weight% of the surface-treated filler,
(c) 0.01 to 25 weight% of initiators, retarders and/or accelerators,
(d) 0 to 25 weight% of auxiliary agents,
wherein the percentages are in each case with reference to the total weight of the material and wherein the filler (b) exhibits a refractive index of no =1.49-1.54,
and the viscosity of the polymerizable dental material, at a storage temperature of 20 to 25°C over at least 9 months, exhibits a value of +/-50% of the starting value, measured 24 h after preparation of the polymerizable dental material,
and the dental material exhibits a reactivity such that, after the start of polymerization, the amount of the maximum heat flux generated by the dental material amounts to at least 0.8 mW/mg and this maximum heat flux is achieved within at most 60 s.

15. Polymerizable dental material prepared according to a process according to Claims 10 to 14.

16. Use of a polymerizable dental material according to one of Claims 1 to 9 or 15 as dental filling material, bonding material or fixing material.

## Revendications

1. Pâte dentaire polymérisable, contenant
(a) 3 à 80 % en poids d'un ou de plusieurs monomères durcissables par polymérisation cationique,
(b) 3 à 90 % en poids d'une ou de plusieurs charges opaques aux rayons X,
(c) 0,01 à 25 % en poids d'amorceurs, de retardateurs et/ou d'accélérateurs,
(d) 0 à 25 % en poids d'adjuvants,
les pourcentages en poids se rapportant respectivement au poids total de la pâte et la charge (b) étant préparée au moyen d'un procédé par fusion à 1550 °C au moins et est choisie de manière à contenir au moins 65 % en poids de SiO₂ et à présenter un indice de réfraction de n_{D} = 1,49-1,54,
et à ce que la viscosité de la pâte dentaire polymérisable à une température de stockage de 20 à 25 °C pendant au moins 9 mois présente une valeur de ± 50 % de la valeur de départ, mesurée 24 h après préparation de la pâte dentaire polymérisable,
et en ce que la pâte dentaire polymérisable présente une telle réactivité qu'après le démarrage de la polymérisation, le niveau du flux thermique maximal produit par la pâte dentaire, s'élève à au moins 0,8 mW/mg et que ce flux thermique maximum est obtenu en 60 secondes au maximum.

2. Pâte dentaire polymérisable selon la revendication 1, dans laquelle la charge (b) présente un indice de réfraction de n_{D} = 1,50-1,53.

3. Pâte dentaire polymérisable selon la revendication 1 ou 2, dans laquelle la charge (b) contient au moins 65 % en poids de SiO₂, en tout au maximum 35 % en poids d'un ou plusieurs des oxydes des éléments des périodes 5 et/ou 6, en tout au plus 3 % en poids d'un ou plusieurs des oxydes du premier groupe principal et en tout au maximum 3 % en poids des oxydes B₂O₃, Al₂O₃, P₂O₅.

4. Pâte dentaire polymérisable selon l'une quelconque des revendications 1 à 3, dans laquelle la charge (b) contient au moins 65 % en poids de SiO₂, en tout au maximum 35 % en poids d'un ou plusieurs des oxydes, choisis dans le groupe Y₂O₃, oxydes de lanthanide, ZrO₂, HfO₂, Nb₂O₅, Ta₂O₅, In₂O₃, SnO₂, WO₃, en tout au maximum 3 % en poids d'un ou plusieurs des oxydes du premier groupe principal et en tout au maximum 3 % en poids des oxydes B₂O₃, Al₂O₃, P₂O₅.

5. Pâte dentaire polymérisable selon l'une quelconque des revendications 1 à 4, dans laquelle la charge (b) est choisie de préférence de manière à présenter une opacité aux rayons X, qui correspond à un équivalent d'aluminium aux rayons X d'au moins 100 %.

6. Pâte dentaire polymérisable selon l'une quelconque des revendications 1 à 5, dans laquelle la charge (b) est préparée selon un procédé par fusion à haute température.

7. Pâte dentaire polymérisable selon la revendication 6, dans laquelle le procédé par fusion à haute température est réalisé à au moins 1600 °C.

8. Pâte dentaire polymérisable selon l'une des revendications 1 à 7, dans laquelle la charge (b) présente une taille moyenne de grains de 0,1 à 15 µm et une surface spécifique de 1 à 35 m²/g.

9. Pâte dentaire polymérisable selon l'une des revendications 1 à 8, dans laquelle la charge (b) présente une taille moyenne de grains de 1 à 4 µm et une surface spécifique de 3 à 9 m²/g.

10. Procédé pour la préparation d'une pâte dentaire polymérisable, dans lequel on prépare la charge au moyen d'un procédé par fusion à haute température à au moins 1550 °C et on l'introduit dans une matrice, contenant
(i) des monomères durcissables par polymérisation cationique,
(ii) des amorceurs, des retardateurs, des accélérateurs et/ou des adjuvants,
la charge contenant au moins 65 % en poids de SiO₂.

11. Procédé pour la préparation d'une pâte dentaire polymérisable selon la revendication 10, dans lequel
(a) on prépare la charge à partir des composants individuels au moyen d'un procédé par fusion,
(b) on broie la charge fondue à une taille de grains de 0,1 à 15 µm
(c) on traite en surface la charge obtenue en (b) et
(d) on introduit la charge traitée en surface dans une matrice, contenant
(i) des monomères durcissables par polymérisation cationique,
(ii) des amorceurs, des retardateurs, des accélérateurs et/ou des adjuvants.

12. Procédé selon l'une quelconque des revendications 10 à 11 dans lequel on réalise le procédé par fusion à une température d'au moins 1600 °C.

13. Procédé selon l'une quelconque des revendications 10 à 12, dans lequel on réalise le procédé par fusion dans un four chauffé par induction à 1550-3000 °C en 5 à 60 minutes ou dans un appareil de fusion au plasma à 2000-3000 °C.

14. Procédé selon l'une quelconque des revendications 11 à 13, dans lequel on transforme la substance traitée en surface en une pâte dentaire contenant
(a) 3 à 80 % en poids d'un ou de plusieurs monomères durcissables par polymérisation cationique,
(b) 3 à 90 % en poids de la charge traitée en surface,
(c) 0,01 à 25 % en poids d'amorceurs, de retardateurs et/ou d'accélérateurs,
(d) 0 à 25 % en poids d'adjuvants,
les indications en pour cent se rapportant à la masse totale de la pâte et la charge (b) présentant un indice de réfraction de n_{D} = 1,49-1,54,
et la viscosité de la pâte dentaire polymérisable à une température de stockage de 20 à 25 °C pendant au moins 9 mois présente une valeur de ± 50 % de la valeur de départ, mesurée 24 h après préparation de la pâte dentaire polymérisable,
et dans lequel la pâte dentaire présente une telle réactivité, qu'après le démarrage de la polymérisation, le niveau du flux thermique maximum produit par la pâte dentaire s'élève à au moins 0,8 mW/mg et ce flux maximum est obtenu en 60 secondes au maximum.

15. Pâte dentaire polymérisable, préparée selon un procédé selon les revendications 10 à 14.

16. Utilisation d'une pâte dentaire polymérisable selon l'une quelconque des revendications 1 à 9 ou 15 comme matériau de plombage dentaire, matériau de collage ou matériau de fixation.
